# NEUE EUROPÄISCHE PATENTSCHRIFT

(11) **EP 0 542 967 B2**
(45) Veröffentlichungstag und Bekanntmachung des Hinweises auf die Entscheidung über den Einspruch: **14.03.2001**
(45) Hinweis auf die Patenterteilung: 15.10.1997
(21) Anmeldenummer: 92911710.9
(22) Anmeldetag: 11.06.1992
(51) Int. Cl.: C11B 7/00

(54) **VERFAHREN ZUR HERSTELLUNG EINES VON BEI DER WEITERVERARBEITUNG STÖRENDEN BESTANDTEILEN BEFREITEN TRIGLYCERID-GEMISCHES**
PROCESS FOR PRODUCING A TRIGLYCERIDE MIX FREE OF COMPONENTS HAMPERING FURTHER PROCESSING
PROCEDE DE PREPARATION D'UN MELANGE DE TRIGLYCERIDES DEPOURVU DE COMPOSANTS GENANT SON TRAITEMENT ULTERIEUR

(30) Priorität: 12.06.1991 DE 4119269; 24.07.1991 DE 4124504
(43) Veröffentlichungstag der Anmeldung: 26.05.1993
(73) Patentinhaber: Dr. Frische GmbH, D-63755 Alzenau (DE)
(72) Erfinder: FRISCHE, Rainer, D-6000 Frankfurt (DE); SCHNEIDER, Judith, D-6238 Hofheim (DE); JÄGER, Erika, D-2000 Hamburg 50 (DE); VOLKHEIMER, Jürgen, D-6200 Wiesbaden (DE); KRÄMER, Michaela, D-5429 Allendorf (DE)
(74) Vertreter: Lippert, Marianne
(86) Internationale Anmeldenummer: EP9201303
(87) Internationale Veröffentlichungsnummer: WO9222626

(56) Entgegenhaltungen:
- EP-A- 0 326 198
- EP-A- 0 367 877
- GB-A- 750 045
- GB-A- 848 689
- US-A- 4 297 292
- DATABASE WPIL Week 9120, Derwent Publications Ltd., London, GB; AN 91-146244

## Beschreibung

Die Erfindung betrifft ein Verfahren zur Herstellung eines von bei der Weiterverarbeitung störenden Bestandteilen befreiten Triglycerid-Gemisches oder von zumindest technisch reinem Triglycerid einheitlicher Fettsäuren aus einem Pflanzenöl und bzw. oder einem das unveränderte Kohlenstoffgerüst eines Pflanzenöls enthaltenden Derivat als Ausgangssubstanz, die als solche oder in Lösung abgekühlt und anschließend in eine überwiegend feste und in eine überwiegend flüssige Phase aufgetrennt wird, worauf das Abkühlen und Auftrennen einer der Phasen oder beider Phasen gegebenenfalls wiederholt werden.

Bei der Weiterverarbeitung von Triglycerid-Gemischen, insbesondere bei der Derivatisierung der Doppelbindung des Ölsäureanteils von derartigen Gemischen, ist es von besonderer Bedeutung, daß das zu verarbeitende Gemisch keine Bestandteile enthält, die Konkurrenzreaktionen zu der Reaktion der Doppelbindung des Ölsäureanteils eingehen. Andererseits ist es nicht erforderlich, zu diesem Zweck den Ölsäureanteil völlig zu isolieren, was zu unvertretbar hohen Kosten führen würde.

Die überwiegende Anzahl der Pflanzenöle, insbesondere die agrarisch produzierten Pflanzenöle, enthalten als Fettsäuren solche mit Kettenlängen von C16 und C18, wobei die am häufigsten vertretenen Fettsäuren die Palmitinsäure (C16), die Stearinsäure (C18), die Ölsäure (C18:1), die Linolsäure (C18:2) und die Linolensäure (C18:3) sind. Die Ziffern hinter dem Doppelpunkt geben die Anzahl der Doppelbindungen in der Fettsäurekette wieder.

Obwohl die Mehrzahl der gängigen Fette und Öle hauptsächlich aus Triglyceriden dieser 5 Fettsäuren bestehen, weisen sie z.T. erhebliche Unterschiede im chemischen und physikalischen Verhalten auf. Diese Unterschiede ergeben sich vor allem daraus, daß selbst bei nur mäßig unterschiedlichen Fettsäuremustern gravierende Unterschiede in der Verteilung der einzelnen Triglyceride in den jeweiligen Ölen und Fetten vorhanden sind.

Bei einem Öl oder Fett mit einem Gehalt von 50 % einer dominierenden Fettsäure im Fettsäuremuster sind nach den Regeln der Kombinatorik über 150 chemisch unterschiedliche Triglyceride in merklichem Anteil vorhanden, wenn alle der oben erwähnten 5 Fettsäuren in merklichem Anteil im Fettsäuremuster vorkommen. Die "Hauptkomponente" ist das lediglich die dominierende Fettsäure tragende Triglycerid. Es ist jedoch nur mit 12,5 % der Gesamttriglyceridmenge vertreten. Dagegen besteht ein Öl oder Fett, in dem eine Fettsäure im Fettsäuremuster mit etwa 80 % vertreten ist, praktisch nur aus solchen Triglyceriden, in denen die dominierende Fettsäure mindestens zweifach vorkommt. Insgesamt besteht ein derartiges Öl zu weit mehr als 90 % aus lediglich neun unterschiedlichen Triglyceriden, wenn alle oben erwähnten fünf Fettsäuren in merklichem Anteil im Fettsäuremuster vorhanden sind. Über 50 % eines derartigen Fettes und Öls wird von dem ausschließlich die dominierende Fettsäure tragenden Triglycerid gebildet.

Aus dem oben Gesagten wird deutlich, daß - wenn überhaupt - eine Abtrennung chemisch einheitlich zusammengesetzter Triglyceride eher bei solchen Ölen und Fetten möglich ist, in denen eine einzelne Fettsäure stark dominiert.

Aber auch für diesen Fall ging die Fachwelt bisher davon aus, daß die Unterschiede im physikalischen Verhalten der jeweiligen Triglyceride so gering sind, daß eine Trennung der Triglyceride über eine einfache physikalische Trennmethode nicht möglich sein würde.

Die Gewinnung chemisch reiner Olsäure, Linolsäure und Linolensäure sowie die Herstellung chemisch reiner Mono-, Di- und Triglyceride dieser Fettsäuren geschieht deshalb bis heute über den Einsatz äußerst aufwendiger physikalischer und chemischer Methoden, wie z.B. die Hochdruckflüssigkeitschromatographie (HPLC) und die chemische Synthese der Mono-, Di- und Triglyceride aus der zuvor chemisch rein dargestellten entsprechenden Fettsäure. Damit lassen sich zwar hochangereicherte, nur einen Fettsäuretyp enthaltende Triglyceride aus Pflanzenölen bzw. deren Derivaten herstellen, aber nur zu ganz erheblichen Kosten. Diese liegen z.B. in der Größenordnung von ca. 10.000.--pro kg für hochreines Trioleat. Bei der derzeit praktizierten Herstellung der reinen Fettsäuren wie auch der reinen Mono-, Di- und Triglyceride übersteigt also der Aufwand für Trennung und Synthese die Kosten für den Rohstoff derart, daß letztere praktisch bedeutungslos sind. Chemische reine reaktive Fettsäuren spielen daher - obwohl preiswerte Rohstoffe mit hohen Gehalten der jeweiligen Fettsäuren vorhanden sind und obwohl die oben erwähnten Fettsäuren von ihrem chemischen Aufbau her als Chemiegrundstoff sehr geeignet sind(z.B. für die Ozonolyse) - im industriellen technischen Bereich wegen der bisher fehlenden Möglichkeit, sie preiswert chemisch rein zu erzeugen, keine Rolle.

Im folgenden wird die Erfindung am Beispiel von solchen Triglycerid-Gemischen beschrieben, die Ölsäure als einen wesentlichen Bestandteil enthalten. Die Erfindung läßt sich jedoch auch auf andere Triglycerid-Gemische analog anwenden.

Ölsäure stellt einen der wichtigsten Rohstoffe der Oleochemie insgesamt dar. Sie kommt in pflanzlichen und tierischen Ölen und Fetten, vergesellschaftet mit einer Vielzahl anderer gesättigter und ungesättigter, zum Teil mehrfach ungesättigter Fettsäuren unterschiedlichster Kettenlängen, vor allem als Triglycerid vor. In der Regel, insbesondere bei den technisch-wirtschaftlich interessanten Fetten und Ölen, dominieren dabei die Fettsäuren mit Kettenlängen zwischen C₁₂ und C₂₂, wobei die C₁₈-Fettsäuren wiederum besonders stark vertreten sind.

Obwohl eine Vielzahl von Verfahren zur Umsetzung der Ölsäure in Folgeprodukte industriell eingesetzt wird - eine der wichtigsten Umsetzungen der Ölsäure ist dabei die Ozonolyse, bei der die Ölsäure in Azelain- und Pelargonsäure oxidativ gespalten wird -, wird als Rohstoff für die Ölsäurechemie nicht chemisch reine Ölsäure, sondern lediglich angereicherte Ölsäure eingesetzt. In der Regel beträgt die Konzentration der Ölsäure im Ronstoff 72 bis 75 %. Gewonnen werden derartige Anreicherungsfraktionen aus preiswertem Talg. Es hat sich dabei gezeigt, daß ohne Verzicht auf den Preisvorteil des Rohstoffs Talg eine höhere Anreicherung als 72 bis 75 % Ölsäuregehalt im Folgeprodukt nicht durchführbar ist. Der dabei ausgenutzte Anreicherungsmechanismus reichen zudem ebenfalls eventuell vorhandene mehrfach ungesättigte Fettsäuren überproportional mit an.

Aufgabe der Erfindung ist daher die Schaffung eines Verfahrens, bei dem einmal Rohmaterial für die Weiterverarbeitung von Triglycerid-Gemischen, insbesondere für die Derivatisierung des Ölsäureanteils solcher Gemische, erzeugt wird, die den interessierenden Bestandteil stärker anqereichert enthalten und die außerdem kaum Bestandteile, die bei der Weiterverarbeitung stören, insbesondere keine oder kaum mehrfach ungesättigte Fettsäuren enthalten. Des weiteren soll ein Verfahren geschaffen werden um zumindest technisch reines und vorzugsweise hochreines, nur einen Fettsäuretyp enthaltendes Triglycerid. wie z.B. Trioleat, aus Pflanzenölen bzw. deren Derivaten als Ausgangssubstanzen kostengünstig herstellen zu können.

Neuerdings werden Ölfrüchte gezüchtet, in deren Ölen die Ölsäure mit Gehalten oberhalb von etwa 80% bereits nativ vorhanden ist ("High-oleic-Öle"). Die Durchführung von Reaktionen an der Ölsäure wird damit wirksam erleichtert.

Die angegebene Grenze von 80 % ist nicht als starr anzusehen; so läßt sich Sonnenblumenöl mit einem Ölsäuregehalt von wenig über 78 % ebenfalls als High-oleic-Öl bezeichnen.

Überraschenderweise hat sich nun außerdem gezeigt, daß es - von derartigen High-oleic-Ölen ausgehend - möglich ist, auf einfache Weise das Ausgangsol in eine Fraktion aufzuteilen, in der nur gesättigte Fettsäuren und Ölsäure enthalten sind, wobei der Gehalt an Ölsäure in dieser Fraktion gegenüber der Ausgangskonzentration steigt, und in eine andere Fraktion, in der die mehrfach ungesättigten Fettsäuren neben Ölsäure enthalten sind, wobei in dieser Fraktion der Gehalt der Ölsäure sinkt. Mit dieser Aufteilung ist es nun möglich vor Durchführung von chemischen Reaktionen an der Doppelbindung der Ölsäure die ebenfalls reagierenden und damit störenden, mehrfach ungesättigten Fettsäuren schon auf der Stufe der Triglyceride abzutrennen.

Gegenstand der Erfindung ist ein zur Herstellung eines von bei der Weiterverarbeitung störenden Bestandteilen befreiten Triglycerid-Gemisches oder von zumindest technisch reinem, nur einen Fettsäuretyp enthaltendem Triglycerid aus einem Pflanzenöl und/oder einem das unveränderte Kohlenstoffgerüst eines Pflanzenöls enthaltenden Derivat als Ausgangssubstanz, die als solche oder in Lösung abgekühlt und anschließend in eine überwiegend feste und in eine überwiegend flüssige Phase aufgetrennt wird, worauf das Abkühlen und Trennen gegebenenfalls wiederholt werden. Das Verfahren ist dadurch gekennzeichnet, daß man
a) als Ausgangssubstanz ein Pflanzenöl einsetzt, in dem eine bestimmte reaktive Fettsäure im Fettsäuremuster mit einem Gehalt von wenigstens etwa 80 % dominiert, daß
b) die Abkühltemperatur eines ersten oder eines einzigen Abkühlschritts so eingestellt wird, daß die besonders schwer erstarrenden bzw. gut löslichen Triglyceride der Ausgangssubstanz, die ,n der Regel mehr reaktive Gruppen enthalten, zumindest im wesentlichen flüssig bzw. in Lösung bleiben, daß
c) gegebenenfalls die nach dem ersten Trennschritt erhaltene feste Phase anschließend gelöst und in einem zweiten Abkühlschritt auf eine von der Temperatur im Schritt b) verschiedene Temperatur abgekühlt wird, bei der die leichter erstarrenden bzw. weniger löslichen und weniger reaktiven Gruppen tragenden Triglyceride zumindest im wesentlichen ausfallen und daß
d) in jedem Fall die zuletzt erhaltene feste und flüssige Phase getrennt und verwertet werden, ggf. nachdem die erste und bzw. oder die beiden Abkühl- und Trennschritte ein- oder mehrmals wiederholt wurden.

Gemäß dem erfindungsgemäßen Verfahren werden also statt üblicher Pflanzenöle bzw. deren Derivaten als Ausgangssubstanzen solche Pflanzenöle verwendet, in denen eine reaktive Fettsäure im Fettsäuremuster zu mindestens etwa 80% dominiert. Bei dem anschließenden Trennverfahren bleibt diese Dominanz erhalten. Ggfs. wird erfindungsgemäß durch eine in zwei aufeinanderfolgenden Abkühl- und Trennschritten b) und c) bei unterschiedlicher Temperatur erfolgende alternierende Abtrennung - beim ersten Trennschritt wird die feste Phase verwendet und beim zweiten Trennschritt die flüssige Phase - der Wirkungsgrad des Abtrennens fühlbar erhöht. Dadurch kann zumindest technisch reines, nur einen einzigen Fettsäuretyp enthaltendes Triglycerid erhalten werden.

Als reaktive Fettsäuren sind beispielsweise Doppelbindungen oder Epoxygruppen oder aus Epoxygruppen durch Ringöffnung erzielbare funktionelle Gruppen oder Gruppenkombinationen enthaltende Fettsäuren bezeichnet.

Entgegen üblichen mehrstufigen Trennverfahren wählt man die Abkühltemperatur des ersten Abkühlschritts niedriger als diejenige des zweiten Abkühlschritts. Dadurch erfolgt eine Anpassung an die Forderung, die Abkühltemperatur des ersten Abkühlschritts so zu wählen, daß (nach Berechnung) der nicht einheitlich zusammengesetzte und leichter lösliche Teil der Triglyceride in Lösung bleibt, während die Abkühltemperatur des zweiten Abkühlschritts so gewählt wird, daß der nicht einheitlich zusammengesetzte und schwerer lösliche Teil der Triglyceride ausfällt.

Auch die flüssige Phase des ersten Trennschritts kann abgetrennt und wie im übrigen auch die feste Phase des zweiten Trennschritts ohne Einschränkung verwertet werden.

Entscheidend für die erfindungsgemäße Trennmethode sind weniger das eingesetzte Lösemittel, das Verhältnis von Lösemittel zu Öl und die bei der Fraktionierung eingehaltenen Temperaturniveaus als vielmehr, über die Wahl des Lösemittels, des Mischungsverhältnisses von Lösemittel zu Fett bzw, Öl und des Temperaturintervalls eine gute Phasentrennung so zu erreichen, daß eine hinreichend einfache Abtrennung der Phasen voneinander möglich ist und die beim Ausfrieren sich bildenden jeweiligen Phasen die vom Fettsäuremuster abhängigen Fraktionsmengen enthalten.

Geht man beispielsweise von einem High-oleic-ÖI aus, das 80 % Ölsäure im Fettsäuremuster hat, so enthält dieses Öl rd. 50 % Glycerintrioleat. Geht man weiter davon aus, daß die gesättigten Fettsäuren ebenso wie die mehrfach ungesättigten Fettsäuren jeweils 10 % (80 + 10 + 10 = 100) im Fettsäuremuster ausmachen, so bestehen die restlichen 50 % des Öls zu jeweils 25 % aus Triglyceriden, die stärker gesättigt sind als das Trioleat, und 25 %, die ungesättigter sind als das Trioleat. Es gilt damit, den ersten Fraktionierungsschritt so zu legen, daß 75 % des Ursprungsöls als Festphase ausfällt und 25 % des Öls in der Flüssigphase verbleiben. Würde man nun diese Fraktionierung lösemittelfrei versuchen, so wäre es technisch praktisch unmöglich, eine Flüssigphase von der ausfallenden Festphase - obwohl sie vorhanden ist - abzutrennen. Um diese Abtrennung zu erleichtern, wird ein Lösemittel verwendet, in dem das Pflanzenöl hinreichend gut löslich ist und aus dem eine hinreichend schnelle Festphasenabtrennung erreicht werden kann. Als geeignete Lösemittel kommen dabei z.B. in Frage Aceton, Methylethylketon, Essigester sowie Mischungen aus diesen Lösemitteln. Das Mischungsverhältnis Öl zu Lösemittel ist dabei im Prinzip in weiten Grenzen frei wählbar.

Verständlicherweise ist die Abtrennung einer kristallinen Festphase von einer Flüssigphase dann besonders gut, wenn der Anteil der Flüssigphase und damit der Lösemittelanteil besonders hoch ist. Wird nämlich die Festphase abfiltriert, so haftet an der kristallinen Festphase zwar noch Restflüssigphase; da aufgrund der hohen Verdünnung jedoch nur wenig Pflanzenöl in dieser enthalten ist, ist die Trennwirkung besonders hoch. Soll andererseits aus einer verdünnten Lösung von Pflanzenöl in Lösemittel die Hauptmenge - in vorliegendem Fall 75% des eingesetzten Öls - als Festphase abgetrennt werden, so benötigt dies sehr tiefe Temperaturen, wodurch wiederum der Aufwand für die Trennung erhöht und das "handling" erschwert wird. Weiterhin hängt die Löslichkeit der unterschiedlichen Triglyceride stark von der Art des Lösemittels ab; so läßt sich durch Wahl des Lösemittels der bei der jeweils zur Fraktionierung benutzten Temperatur lösliche Anteil der Triglyceride verändern, wie weiter unten aus den Ausführungsbeispielen ersichtlich.

Welches Lösemittel, welche Fraktionierungstemperatur und welcher Verdünnungsgrad letztlich gewählt werden, hängt von der anhand des Fettsäuremusters zu berechnenden Triglyceridverteilung sowie von den apparativen Möglichkeiten ab.

Die Gewinnung von Trioleat aus High-Oleic-ölen kann z.B. sowohl dadurch erfolgen, daß zunächst mehrfach bei -27°C aus einer 3:1 Aceton/High-Oleic-Öl-Mischung die Festphase von der Flüssigphase getrennt wird. Die Flüssigphasen enthalten dann angereichert die linolen- und linolsäurehaltigen Triglyceride. (Sie können nicht auf einmal abgetrennt werden, da bei -27°C die abzutrennende Triglyceridmenge in der Lösemittelmenge nicht gut genug löslich ist. Wird bei gleicher Verdünnung die Trennung bei -20°C durchgeführt, so enthält die Flüssigphase zwar mehr abzutrennendes Triglycerid, die am Kristallbrei (Festphase) haftende Flüssigphase ist jedoch konzentrierter, so daß des isolierte Festphasenmaterial zur Erreichung einer Trennung noch einmal fraktioniert werden muß.) Anschließend wird das bei -27°C als Festphase anfallende Öl wiederum 3:1 mit Aceton gelöst und bei -5°C mehrfach fraktioniert.

Die bei -5°C ausfallenden Festphasen enthalten stark angereichert die die gesättigte Fettsäure tragenden Triglyceride, wohingegen das Trioleat in der Flüssigphase verbleibt.

Als besonders vorteilhaft hat es sich bei dieser Trennung erwiesen, die Fraktionierung bei ca. -27°C (Tiefkühltruhe) und bei ca. -5° bis -10°C alternierend durchzuführen. Auf diese Weise wird erreicht, daß das Verhältnis der im Vergleich zum Trioleat besser löslichen Triglyceride zu den schlechter löslichen Triglyceriden bei Wiederholung der Fraktionierungsschritte nicht zu sehr auf eine Seite hin verschoben wird. Dies wäre zwar bei einer klassischen Fraktionierung wünschenswert, führt jedoch bei Triglyceriden offensichtlich dazu, die Ausbeute an zu gewinnendem Trioleat zu verringern.

Dies zeigt, daß die Fraktionierung von Triglyceriden nicht einfachen, mathematisch beherrschbaren Regeln der physikalischen Chemie - wie sie üblicherweise für Fraktionierungsoperationen ansetzbar sind - unterliegen. Vorstellbar ist z.B., daß der Gehalt an Trioleat in den abzutrennenden Phasen (im Vergleich zum Trioleat besser lösliche Triglyceride und im Vergleich zu dem Trioleat schlechter lösliche Triglyceride vom Gehalt an Nicht-Trioleatbestandteilen in der Trioleatphase abhängt. Hierfür spricht, daß die aus einer Aceton-High-Oleic-ÖI-Mischung (3:1) gewinnbare Phase der besser löslichen Triglyceride stets auch Gehalte an schlechter löslichen Triglyceriden und Trioleat aufweist. Dies ändert sich auch nicht, wenn die Temperatur weiter gesenkt wird, vielmehr nimmt dann sehr stark die Gesamtmenge der löslichen Triglyceride ab. Gleiches gilt entsprechend für die Fraktion der schlechter löslichen Triglyceride.

Die Gewinnung von Trilinoleat bzw. Trilinolenat ist im Prinzip ebenfalls durch Fraktionierung entsprechender, an Linolsäure bzw. Linolensäure reicher Öle möglich. Öle mit Gehalten von ≥ 80% dieser Säuren im Fettsäuremuster sind jedoch praktisch nicht vorhanden.

Normales Sonnenblumenöl und Distelöl enthält jedoch ca. 75% Linolsäure im Fettsäuremuster. Aus diesen Ölen läßt sich mit dem erfindungsgemäßen Verfahren Trilinoleat zumindest sehr stark angereichert in hoher Ausbeute - allerdings bei deutlich veränderten Trennbedingungen (anderes Temperaturintervall und/oder andere Lösemittel) gewinnen.

Die Berechnung der Ausbeute darf dabei nur auf das tatsächlich im Ausgangsöl vorhandene Trilinoleat bezogen werden. Dies ist bei 75% Linolsäure im Fettsäuremuster des Ausgangsöls ca. 42% der Gesamtmenge des Ausgangsöls.

Werden speziell auf die Reaktion von Doppelbindungen ausgelegte chemische Operationen mit der nach dem Verfahren gemäß der Erfindung erhaltenen, die Ölsäure tragenden Fraktion durchgeführt, so reagiert hierbei lediglich die Ölsäure, da die anderen ungesättigten Fettsäuren fehlen. Nicht reagieren natürlich auch die gesättigten Fettsäuren. Dabei ist es unbedeutend, ob diese Reaktion am Triglycerid selbst oder an Folgeverbindungen durchgeführt wird, etwa den aus den Triglyceriden gewinnbaren Fettsäuren und Fettsäureestern. Zu derartigen Folgeverbindungen zählen insbesondere die Fettsäuren, die Methyl- und Ethylester der Fettsäuren sowie Amide, Diamide der Fettsäuren und Fettalkohole.

Ein Vorteil der Erfindung besteht auch darin, daß mit der Derivatisierung der Ölsäure in dem erfindungsgemäß erhaltenen Produkt die Abtrennung der durch Umsetzung der Doppelbindung zu Derivaten entstehenden Folgeverbindungen der Ölsäure erheblich erleichtert wird. So lassen sich die gesättigten Fettsäuren - in der Regel handelt es sich um Palmitin- und Stearinsäure - wesentlich einfacher von derartigen Derivaten und/oder Folgeprodukten der Ölsäure abtrennen als von der Ölsäure selbst. Die sei am Beispiel der Ozonolyse dargelegt, bei der letztlich Palmitin und Stearinsäure von den Ölsäurefolgeprodukten abgetrennt werden.

Wird die erfindungsgemäß gewonnene Ölsäurefraktion als Rohstoff für die Ozonolyse eingesetzt, so wird hierbei deren Überlegenheit gegenüber anderen vergleichbaren Rohstoffen besonders deutlich:
1) Der Ozonverbrauch wird ausschließlich von der umzusetzenden Ölsäure bestimmt und nicht - wie derzeit der Fall - auch von anderen, insbesondere mehrfach ungesättigten Fettsäuren. Gerade die mehrfach ungesättigten Fettsäuren haben einen besonders hohen Ozonverbrauch, weil pro Doppelbindung ein Ozonäquivalent verbraucht wird. Hierbei ist zu berücksichtigen, daß der Ozonverbrauch bei der Ozonolyse der kostenintensivste Faktor des Verfahrens ist.
2) Da außer der Ölsäure keine andere mit Ozon reagierende Fettsäure vorhanden ist, entstehen bei der Ozonolyse auch keine Folgeprodukte, wie sie üblicherweise aus den derzeit für die Ozonolyse eingesetzten Rohstoffen zwangsweise entstehen. So entstand bisher aus Linolsäure zwar auch Azelainsäure als Dicarbonsäure wie bei der Ölsäure, jedoch darüber hinaus auch Hexansäure und Malonsäure. Diese beiden Verbindungen erschweren die Gewinnung der gewünschten Produkte der Ozonolyse Pelargonsäure und Azelainsäure. Analog entstanden aus Linolsäure Propionsäure, Malonsäure und Azelainsäure. Diese unerwünschten Nebenreaktionen erhöhten den Aufwand und die Kosten für die Ozonolyse erheblich.
3) Die erfindungsgemäß gewonnene Ölsäurefraktion aus High-oleic-Ölen enthält neben Ölsäure auch andere gesättigte Fettsäuren. Dieser Gehalt an gesättigten Fettsäuren ist für die Ozonolyse nicht störend, im Gegenteil: da die Ozonolyse zur Vermeidung zu hoher Ozonidkonzentrationen ohnehin flüssige, gesättigte Fettsäuren - in der Regel Pelargonsäure - enthält, sind die in der erfindungsgemäß gewonnenen Ölsäurefraktion vorhandenen gesättigten Fettsäuren reaktionserleichternd: die Pelargonsäure als gewünschtes Folgeprodukt der Ölsäure-Ozonolyse kann einfach in ihnen abgetrennt werden, die gesättigten Fettsäuren selbst jedoch übernehmen die notwendige Funktion als Lösemittel für die Reaktion.

Das erfindungsgemäße Verfahren erleichtert damit erheblich die Umsetzung und die Gewinnung von Folgeprodukten und Derivaten aus Ölsäure, die durch Umsetzung der Ölsäure an der Doppelbindung entstehen.

Das erfindungsgemäße Verfahren zur Gewinnung von lediglich Ölsäure als ungesättigte Fettsäure enthaltendem Rohstoff verwendet als Ausgangsmaterial High-oleic-Öle, d. h. native Öle mit einem Gehalt von mindestens 80 % Ölsäure im Fettsäuremuster: sie können von unterschiedlichster Qualität sein. Als Rohstoff für das erfindungsgemäße Verfahren eignen sich rohe, lediglich über Auspressen von Ölfrüchten gewonnene High-oleic-Öle wie auch hoch raffinierte, von freien Fettsäuren, Schleimstoffen, Farbstoffen und anderen Begleitstoffen befreite High-oleic-Öle. Die Qualität der Einsatzstoffe hat dabei praktisch weder einen negativen Einfluß auf die Durchführbarkeit und Effizienz des Verfahrens selbst, noch unterscheidet sich die gewinnbare Ölsäurefraktion in ihrer Eignung als Rohstoff für Ölsäurederivatisierungsprozesse. Lediglich die die mehrfach ungesättigten Fettsäuren tragende Fraktion weist je nach Einsatzstoff deutlich unterschiedliche Qualitäten auf.

Da es sich bei dem erfindungsgemäßen Verfahren um ein physikalisches Trennverfahren handelt, das keinerlei chemische Veränderung der Ausgangsöle vornimmt, kann die die mehrfach ungesättigten Fettsäuren tragende Fraktion praktisch für alle der Fettsäurezusammensetzung entsprechenden Anwendungen, also auch für Ernährungszwekke, eingesetzt werden.

Unter Kostengesichtspunkten kann es dabei durchaus vorteilhafter sein, die Gewinnung der Ölsäurefraktion, von ungereinigten Ölen ausgehend, vorzunehmen und die dabei anfallende, die mehrfach ungesättigten Fettsäuren enthaltende Nebenfraktion anschließend einem üblichen Reinigungsprozeß zu unterziehen. Auf diese Weise wird zum einen die überwiegende Hauptmenge des Ausgangsmaterials als Ölsäurefraktion abtrennbar, ohne daß bereits für sie aufwendige Reinigungsprozesse durchgeführt werden müssen. Die die Verunreinigungen des Ausgangsmaterials enthaltende Nebenfraktion, die nur einen geringen Anteil des Ausgangsmaterials ausmacht, kann anschließend über übliche Reinigungsoperationen in der Regel insgesamt effizienter und verlustärmer und damit mit erheblich geringerem Aufwand gereinigt werden als bei Reinigung der gesamten Ausgangsmenge.

Angewandt auf die hauptsächlich Ölsäure enthaltenden Triglycerid-Gemische besteht also das erfindungsgemäße Verfahren darin, daß das Ausgangsmaterial auf eine Temperatur abgekühlt wird, bei der lediglich die die mehrfach ungesättigten Fettsäuren enthaltenden Triglyceride flüssig bleiben. Sämtliche anderen Triglyceride, Triglyceride also, die gesättigte Fettsäuren und die Ölsäure enthalten, werden bei dieser Temperatur verfestigt bzw. auskristallisiert.

Das erfindungsgemäße Verfahren beruht auf Erkenntnissen, die sich aus bekannten, empirischen Beobachtungen ergeben, und verknüpft diese mit neuen, lediglich für High-oleic-Öle gültigen und der Fachwelt aufgrund von Vorurteilen nicht zugänglich gewesenen Erkenntnissen. So ist aus Erfahrung bekannt, daß Triglyceride einen um so niedrigeren Erstarrungspunkt besitzen, je höher der Anteil ungesättigter Fettsäuren ist. Der Fachwelt bisher entgangen ist jedoch, daß bei High-oleic-Ölen, selbst bei statistischer Verteilung der Fettsäuren im Triglycerid - anders als bei klassischen Ölen - wenige, chemisch einheitliche Triglyceride die Menge der Öls bestimmen.

Wie weiter oben bereits erwähnt, werden bei einem Ölsäuregehalt von etwa 80%, wie er für High-Oleic-Öle stets erreicht wird, 50% des Pflanzenöls von chemisch einheitlichem Trioleat gebildet. Die restlichen 50% werden dabei zum überwiegenden Teil von Triglyceriden gebildet, die neben einem anderen Fettsäurerest zwei Ölsäurereste im Triglycerid chemisch gebunden enthalten. Triglyceride mit nur einem oder gar keinem Ölsäurerest sind in High-oleic-Ölen - anders als bei klassischen Ölen - mengenmäßig vernachlässigbar. Aus dieser für High-oleic-Öle typischen Erkenntnis läßt sich ableiten, daß die Hauptmenge der mehrfach ungesättigten Fettsäuren im Triglycerid mit zwei Ölsäureresten vergesellschaftet ist und damit bezüglich ihres Erstarrungs- bzw. Ausfrierpunkts unter dem des Trioleats liegen sollte.

Für das Verfahren erleichternd kommt hinzu, daß die Kristallisationsfreudigkeit des Trioleats aufgrund seines hohen Gehalts im Vergleich zu klassischen Ölen deutlich steigen sollte.

Auch die gesättigten Fettsäuren liegen in High-oleic-Ölen als Triglyceride in der Hauptmenge mit jeweils zwei Ölsäuren gebunden vor. Diese Triglyceride sollten damit bezüglich ihres Erstarrungspunkts höher liegen als die entsprechenden Triglyceride, die mehrfach ungesättigte Fettsäuren enthalten.

Nach allgemeinem Verständnis war jedoch zu erwarten, daß trotz dieser prinzipiellen theoretischen Möglichkeit die Trennung der Triglyceride über die Kristallisationsfreudigkeit und den Unterschied im Erstarrungspunkt dennoch eine saubere Trennung nicht möglich ist,
1. weil die Unterschiede zu gering sind, insbesondere wenn man berücksichtigt, daß praktisch in allen Triglyceridmolekülen in High-oleic-Ölen mindestens zwei Ölsäurereste chemisch gebunden vorliegen,
2. weil aufgrund der äußerst ähnlichen chemischen Struktur ein Einschluß und Einbau "falscher" Triglyceride beim Auskristallisieren und Ausfrieren der jeweiligen Phasen erfolgen bzw. quasi eutektische Gemische der Triglyceride entstehen. Mit diesen Argumenten wird häufig begründet, warum eine Reindarstellung von Ölsäure nicht möglich ist.

Überraschenderweise läßt sich nach dem erfindungsgemäßen Verfahren ein die Trennung behinderndes Mitfällen oder E inschließen störender Triglyceride vermeiden, bzw. dieser die Trennung verhindernde Vorgang findet nicht statt.

Das erfindungsgemäße Verfahren zur Abtrennung von lediglich Ölsäure als ungesättigte Fettsäure enthaltenden Triglyceriden aus High-oleic-Ölen kann in klassischen Anlagen, wie sie zur Talgfraktionierung benutzt werden, durchgeführt werden, wobei jedoch das zu fahrende Temperaturprofil auf die geänderte Zielsetzung abgestimmt werden muß. Das Verfahren wird dabei so eingestellt, daß als Flüssigphase letztlich der aus dem Fettsäuremuster der High-oleic-Öle bestimmbare bzw. berechenbare Anteil von Triglyceriden mit mehrfach ungesättigter Fettsäure gewonnen wird. Ein gewisser Sicherheitszuschlag in Höhe von 10 bis 20 %, bezogen auf den Teil der abzuscheidenden, mehrfach ungesättigte Fettsäuren enthaltenden Triglyceridmenge, sollte dabei einkalkuliert werden.

Das erfindungsgemäße Verfahren läßt sich auch unter Zuhilfenahme geeigneter Lösemittel, wie beispielsweise Aceton oder Essigester, durchführen. Der Einsatz von Lösemitteln hat dabei zur Folge, daß wegen des Verdünnungseffekts die Abtrennung der Flüssigphase des Triglycerids wesentlich effizienter pro Fraktionierungsschritt erfolgen und damit die Anzahl der Fraktionsschritte verringert werden kann. Dies ist insbesondere für Batchprozesse bedeutend. Das Lösemittel kann in jedem Fall im Kreislauf geführt werden.

Die Erfindung wird im folgenden anhand von Zeichnungen in Verbindung mit Ausführungsbeispielen näher erläutert. Es bedeuten:
Fig. 1 ein Fließschema für eine fraktionierte Glyceridtrennung in allgemeiner Form gemäß der Erfindung;
Fig. 2 ein Fließschema für die fraktionierte Glyceridtrennung eines High-Oleic-Sonnenblumenöls gemäß der Erfindung, wie sie in Beispiel 2 beschrieben ist; und
Fig. 3 bis Fig. 6 Abbildungen von Dünnschichtchromatogrammen, die die Zusammensetzung einzelner Fraktionen, wie sie bei fraktionierten Glyceridtrennungen gemäß den Beispielen der Erfindung erhalten wurden, wiedergeben.

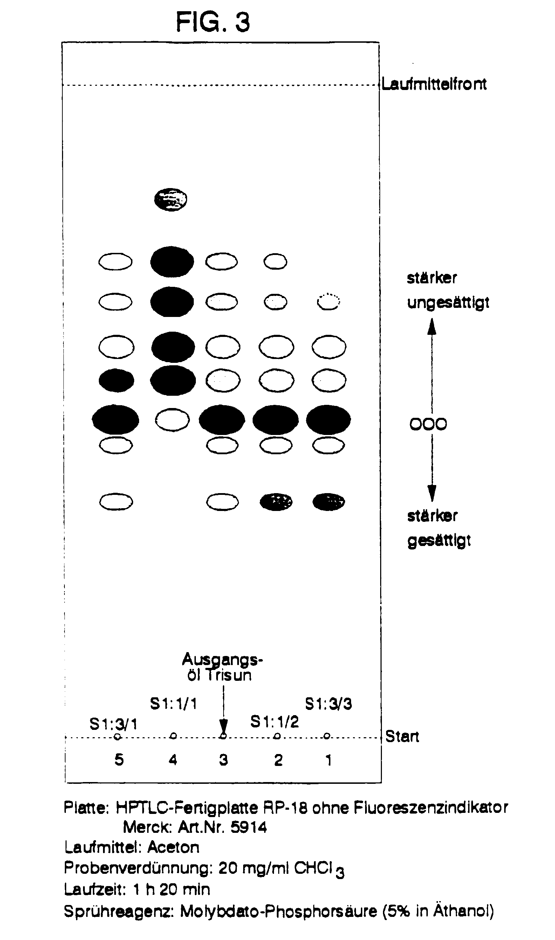

In den Beispielen und den zugehörigen Chromatogrammen (Fig. 3 bis 6) bedeuten:
O = Oleat
L = Linoleat
E = Linolenat,
demnach also 000 = (Glycerin-)trioleat
oder OOL = ( " -)dioleat-monolinoleat

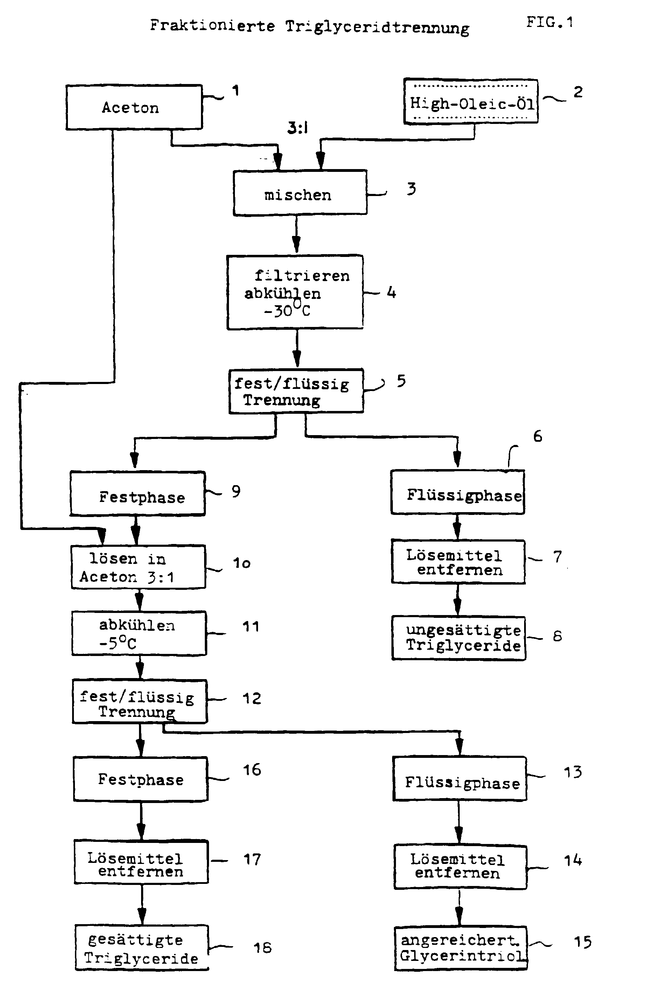

Das Diagramm gemäß Fig. 1 erläutert eine fraktionierte Triglyceridtrennung anhand des erfindungsgemäßen Verfahrens, bei der beide Trennstufen b) und c) verwirklicht sind und das zu reinem Glycerintrioleat führt.

Aceton 1 und ein Pflanzenöl 2 (hier als High-Oleic-Oil bezeichnet), in dem eine reaktive Fettsäure im Fettsäuremuster mit einem Gehalt von wenigstens 80% dominiert, werden bei Pos. 3 in einem Verhältnis von beispielsweise 3: 1 gemischt. Die Mischung wird anschließend gegebenenfalls filtriert (bei rohen Ölen) und auf -30°C abgekühlt, wie durch das Kästchen 4 angedeutet. Anschließend erfolgt die durch das Kästchen 5 symbolisierte Trennung in eine feste und eine flüssige Phase.

Aus der Flüssigphase 6 wird gemäß Kästchen 7 das Lösemittel entfernt, und man erhält die besser löslichen, hauptsächlich nicht einheitlich zusammengesetzten, ungesättigteren Triglyceride, wie bei Pos. 8 angedeutet.

Die Festphase 9 wird in Aceton gelöst, beispielsweise im Verhältnis 3:1, wie im Kästchen 10 angedeutet. Anschließend wird die Lösung auf -5°C abgekühlt. Siehe Pos. 11. Anschließend erfolgt die Trennung in die feste und die flüssige Phase. Siehe Kästchen 12.

Die Flüssigphase des Trennschritts Pos. 12 ist bei Pos. 13 angedeutet, und aus ihr wird das Lösemittel entfernt, wie bei Pos. 14 angedeutet. Man erhält daraus angereichertes Glycerintrioleat, Pos. 15.

Aus der Festphase 16 des Trennschritts Pos. 12 entfernt man das Lösemittel, wie bei Pos. 17 angedeutet, und man erhält daraus die weniger gut löslichen, hauptsächlich nicht einheitlich zusammengesetzten, gesättigteren Triglyceride 18.

Die vorstehend beschriebenen Abkühl- und Trennschritte werden ggf. einmal oder mehrfach wiederholt.

Das Trennverfahren läßt sich analog auf epoxidierte High-Oleic-Öle sowie auf die daraus durch reduktive und/ oder hydrolytische Epoxyringöffnung erhältlichen, pro Epoxygruppe eine (reduktive Öffnung) bzw. zwei Hydroxylgruppen (hydrolytisch) bzw. eine Ketogruppe (Ringöffnung mit Lewis-Säuren) enthaltenen Derivate übertragen.

Analog kann auf diese Weise aus Ricinusöl und aus hydriertem Ricinusöl das Triglycerid der Ricinolsäure bzw. das Triglycerid der 12-Hydroxystearinsäure isoliert werden.

### Beispiel 1

### Fraktionierung von Euphorbiaöl mit Aceton

Rohes High-oleic-Euphorbiaöl (gewonnen über Abpressen von Euphorbia-lathyris-Samenkörnern) wird mit der dreifachen Menge Aceton vermischt. Es entsteht eine trübe, leicht gelblich gefärbte, homogene Lösung. Die Trübungen sind zurückzuführen auf nicht-triglyceridische Verunreinigungen des Preßöls. Sie lassen sich weitestgehend durch Filtration der Aceton-Pflanzenöl-Lösung entfernen.

Die erhaltene filtrierte Lösung wird auf -28°C langsam abgekühlt (über Nacht). Es fällt ein nahezu farbloser Kristallbrei aus, der mit einer vorgekühlten Nutsche scharf abgesaugt wird.

Die Flüssigphase enthält dabei die mehrfach ungesättigte Fettsäuren tragenden Triglyceride, die Festphase im wesentlichen Trioleat und Triglyceride, die neben Ölsäure jeweils eine gesättigte Fettsäure (Palmitin-, Stearinsäure) enthalten.

Des Fettsäuremuster der Festphase zeigt gemäß dem Chromatogramm, daß die mehrfach ungesättigten Fettsäuren (Linol- und Linolensäure) des Euphorbiaöls weitestgehend entfernt wurden. Das Fettsäuremuster der Flüssigphase zeigt, daß die mehrfach ungesättigten Fettsäuren hierin aufkonzentriert wurden. Zu beachten ist hierbei, daß auch die Flüssigphase im Fettsäuremuster vor allem Ölsäure aufweist: dies deshalb, weil die mehrfach ungesättigten Fettsäuren jeweils mit zwei Ölsäuren triglyceridisch im Euphorbiaöl gebunden vorliegen müssen.

Wird die Abtrennungsoperation wiederholt - der feste Rückstand des bei -30°C ausgefallenen Pflanzenöls wird erneut in der dreifachen Menge Aceton aufgelöst und bei -30°C ausgefällt und vom Lösemittel befreit - so läßt sich der Gehalt an mehrfach ungesättigten Fettsäuren in der Feststofffraktion weiter senken. In der Flüssigphase verbleiben dabei verständlicherweise höhere Mengen an Ölsäure in Form von Triglyceriden, als nach der Berechnung des Anteils von Triglyceriden mit mehrfach ungesättigten Fettsäuren abgetrennt werden müssen.

Das Filtrat des Tieftemperatur-Kristallisationsprozesses läßt sich zu einem Öl aufbereiten, das seiner Qualität nach einem stark ungesättigten Pflanzenöl entspricht. Hierzu werden - je nach Qualität des Ausgangsmaterials und der Art des Ausfrierprozesses - eventuell vorhandenes Lösemittel entfernt und die klassischen Operationen zur Reinigung wie bei ungesättigten Pflanzenölen durchgeführt (Entsäuern, Entschleimen, Entfärben, Desodorieren, etc.). Das so gewonnene Nebenprodukt stellt ein gegebenüber dem Ausgangsprodukt zumindest gleichwertiges, wenn nicht höherwertiges Produkt dar, das für eine Vielzahl von Anwendungen eingesetzt werden kann.

### Beispiel 2

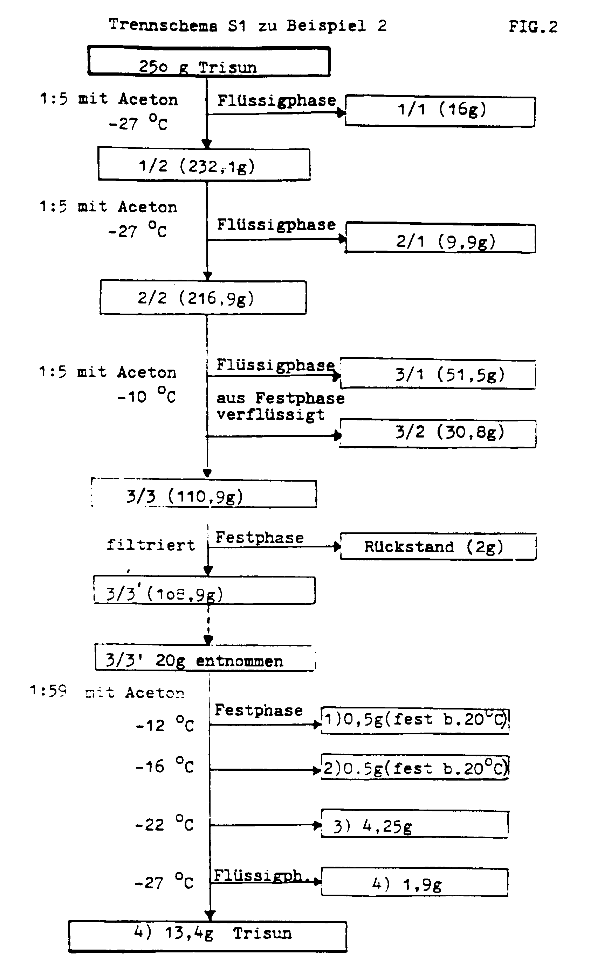

### Fraktionierung von Sonnenblumenöl mit Aceton / vgl.Fig.2

250 g Sonnenblumenöl "TRISUN 80 RBD"; Charge SC 1270, das nach Analyse:
C 18:1 78,55%
C 18:2 10,70%
C 18:3 0,19%
enthält, werden mit 1250 g Aceton gemischt (1:5 Massenanteile). Die Lösung wird über Nacht im Tiefkühlfach bei - 27°C aufbewahrt. Anschließend wird die ausgefrorene Festphase 1/2 über eine vorgekühlte Glasfritte von der Flüssigphase 1/1 getrennt. Bei beiden Phasen wird das Lösemittel Aceton mittels Rotationsverdampfer entfernt.

| | | |
|---|---|---|
| Ausbeuten: | 1/1 Trisun in Flüssigphase | 16 g |
| | 1/2 Trisun in Festphase | 226,5 g |

Dieser Vorgang wird nun noch einmal mit dem Öl aus der Festphase wiederholt: d.h. es wird im Verhältnis 1:5 mit Aceton gemischt und über Nacht im Tiefkühlfach bei -27°C aufbewahrt. Fest- und Flüssigphase werden, wie oben beschrieben, getrennt und die Ausbeuten bestimmt.

| | | |
|---|---|---|
| Ausbeuten: | 2/1 Trisun in Flüssigphase | 9,9 g |
| | 2/2 Trisun in Festphase | 216,9 g |

Das Öl aus der Festphase wird nun wiederum im Verhältnis 1:5 mit Aceton vermischt und über Nacht in einem Kryo-Bad bei -10°C aufbewahrt. Die entstandene Festphase wird nun von der Flüssigphase 3/1 über eine vorgekühlte Glasfritte getrennt. Bei weiterem Stehenlassen der Festphase verflüssigte sich ein Teil dieser Phase, der ebenfalls von der verbleibenden Festphase entfernt wird.

| | | |
|---|---|---|
| Ausbeuten: | 3/1 Trisun in Flüssigphase | 51,5 g |
| | 3/2 Trisun in Festphase (verflüssigt) | 30,8 g |
| | 3/3 Trisun in Festphase | 110,9 g |

Das Chromatogramm in Fig. 3 zeigt einige der Phasen im Vergleich zum Ausgangsöl. Die Flüssigphase 1/1 zeigt eine deutliche Zunahme der Fleckintensität für mehrfach ungesättigte Fettsäuren enthaltende Triglyceride. Festphase 3/3 zeigt eine deutliche Abnahme dieser Intensität.

### Die Analysen zeigen folgende Zusammensetzung für die Flüssigphasen:

| | |
|---|---|
| 1/1 Trisun in Flüssigphase (-27°C): | C16:0 = 3,58% |
| | C18:0= 1,68% |
| | C18:1 = 41,57% |
| | C18:2 = 51,34% |
| | C20:0 = 0,20% |
| | C22:0 = 0,32% |

| | |
|---|---|
| 2/1 Trisun in Flüssigphase (-27°C): | C16:0 = 3,64% |
| | C18:0= 1,70% |
| | C18:1 = 45,21% |
| | C18:2 = 48,52% |

In den nächsten Schritten werden diejenigen Triglyceride abgetrennt, die die gesättigten Fettsäuren enthalten, um so zum Schluß möglichst reines Trioleat zu erhalten.

Phase 3/3 ist milchig-trüb. Sie wird filtriert. Auf dem Filter verbleibt ein grauer Rückstand (2 g).

Von Phase 3/3' (filtriert: klar, hellgelb) werden 20 g entnommen und mit 1,5 Liter (1,185 kg) Aceton vermischt (1: 59 Massenanteile). Die Lösung wird in den Tiefkühlschrank gestellt.
1 ) Nach ca. 2 Stunden bildet sich bei einer Lösungstemperatur von -12°C ein erster voluminöser, flockiger, weißer Niederschlag (sehr wenig). Nach Filtrieren über eine vorgekühlte Glasfritte und Entfernen des Acetons mit einem Rotationsverdampfer verbleibt ein Rückstand von 0,5 g der bei Raumtemperatur fest ist.
2) Die flüssige Phase wird im Tiefkühlschrank weiter abgekühlt. Bei -16°C wird der zweite Niederschlag abfiltriert. Nach Entfemen des Lösemittels verbleibt ein Rückstand von ca. 0,5 g, der bei Raumtemperatur fest ist.
3) Die flüssige Phase wird weiter abgekühlt. Bei -22°C wird der nächste Niederschlag abfiltriert. Nachdem das Aceton mit einem Rotationsverdampfer entfernt wurde, verbleibt ein Rückstand von 4,3 g, der bei Raumtemperatur als Öl vorliegt.
4) Nun wird die Flüssigphase über Nacht bei -27°C im Tiefkühlschrank gelassen. Anschließend wird über eine vorgekühlte Glasfritte filtriert und bei beiden Phasen das Lösemittel entfernt.

| | | |
|---|---|---|
| Ausbeuten: | Trisun in Flüssigphase | 1,9 g |
| | Trisun in Festphase | 13,4 g (entspricht 30% Ausbeute, bezogen auf die Einsatzmenge von 250 g) |

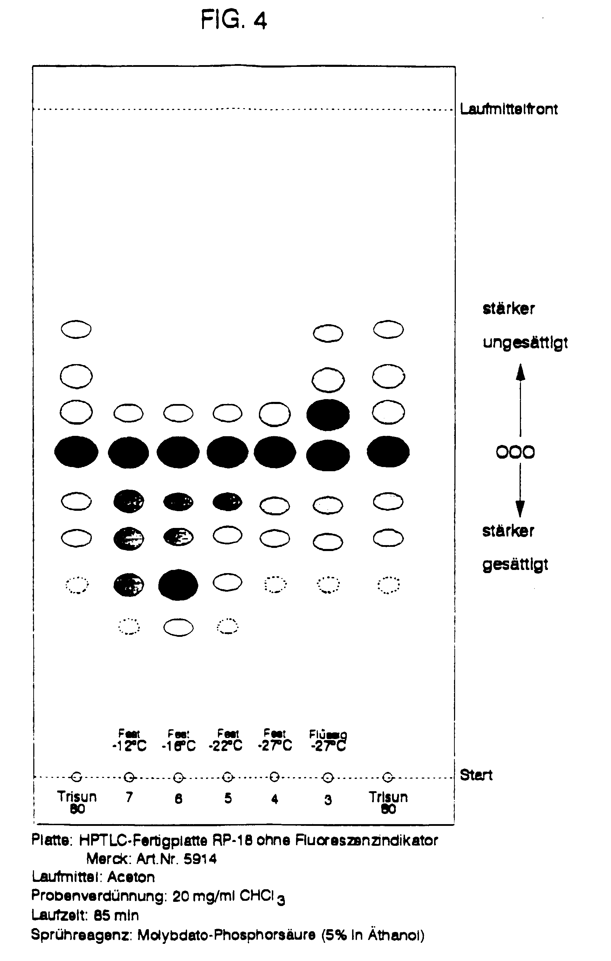

Das Chromatogramm Fig. 4 zeigt diese Phasen im Vergleich zum Ausgangsöl. Die Platte zeigt, daß die Festphasen, die bei -12 u. -16°C abgetrennt wurden, die gesättigten Fettsäuren enthaltende Triglyceride verstärkt enthalten. Bei der zum Schluß bei -27°C abgetrennten Flüssigphase sind die oberen Triglyceridflecken oberhalb 000 deutlich verstärkt auf dem Chromatogramm zu erkennen.

Die bei -27°C erhaltene mit Triolein angereicherte Festphase zeigt einen sehr starken Triolein-Chromatographiefleck, sowie einen schwachen Fleck darüber (LOO) und 1 bis 2 schwache Flecken darunter.

### Die Analysen zeigen folgende Zusammensetzung der Fest-und Flüssigphasen:

| | |
|---|---|
| 1) Trisun in Festphase (-12°C): | C16:0 =12,96% |
| | C18:0 = 23,77% |
| | C18:1 = 42,55% |
| | C18:2 = 3,04% |
| | C20:0 = 2,89% |
| | C22:0 = 9,60% |
| | C24:0 = 3,80% |

| | |
|---|---|
| 2) Trisun in Festphase (-16°C): | C16:0 = 4.10% |
| | C18:0 = 9,59% |
| | C18:1 = 60,94% |
| | C18:2 = 2,29% |
| | C22:0 = 23,09% |

| | |
|---|---|
| 3) Trisun in Festphase (-22°C): | C16:0 = 4,04% |
| | C18:0 = 8,09% |
| | C18:1 = 82,10% |
| | C18:2 = 1,90% |
| | C20:0 = 0,70% |
| | C22:0= 1,95% |
| | C24:0 = 0,77% |

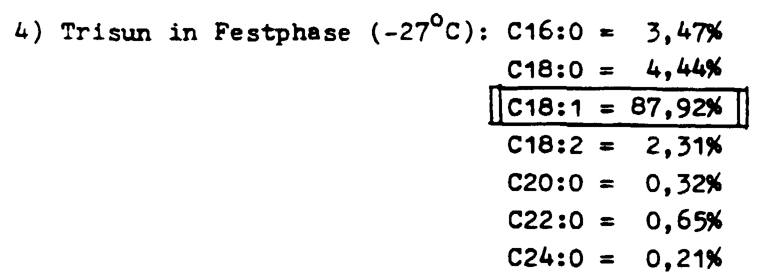

| | |
|---|---|
| Trisun in Flüssigphase (-27°C): | C16:0 = 4,58% |
| | C18:0 = 2,76% |
| | C18:1 =63,16% |
| | C18:2 = 26,92% |
| | C20:0 = 0,21% |
| | C22:0 = 0,82% |
| | C24:0 = 0,30% |

Mit dieser Fraktionierung konnte der Ölsäuregehalt von 78,55% im Ausgangsöl auf 87,92% angereichert werden.

### Beispiel 3

### Fraktionierung von Sonnenblumenöl mit Aceton / Ethyl-methylketon

### Verwendetes Lösemittel:

Aceton : Ethyl-methylketon = 9:1 (Volumen)

### Fraktionierung

5 g Trisun 80 - Öl werden mit 15 g des Lösemittels vermischt (1:3 (Massenanteile)). Bei Raumtemperatur (20°C) löst sich das Öl im Lösemittel. Die Mischung wird nun mehrere Stunden im Tiefkühlschrank bei -27°C aufbewahrt. Die entstandene Festphase wird über eine vorgekühlte Glasfritte durch Absaugen mit einer Wasserstrahlpumpe getrennt. Bei Raumtemperatur verflüssigt sich die Festphase wieder. Bei beiden Phasen wird nun die Lösemittelmischung am Rotationsverdampfer entfernt.

### Ausbeuten:

Ölphase (= Festphase): 4,2 g

Öl in der Lösemittelphase (Flüssigphase): 0,5 g

### Auswertung:

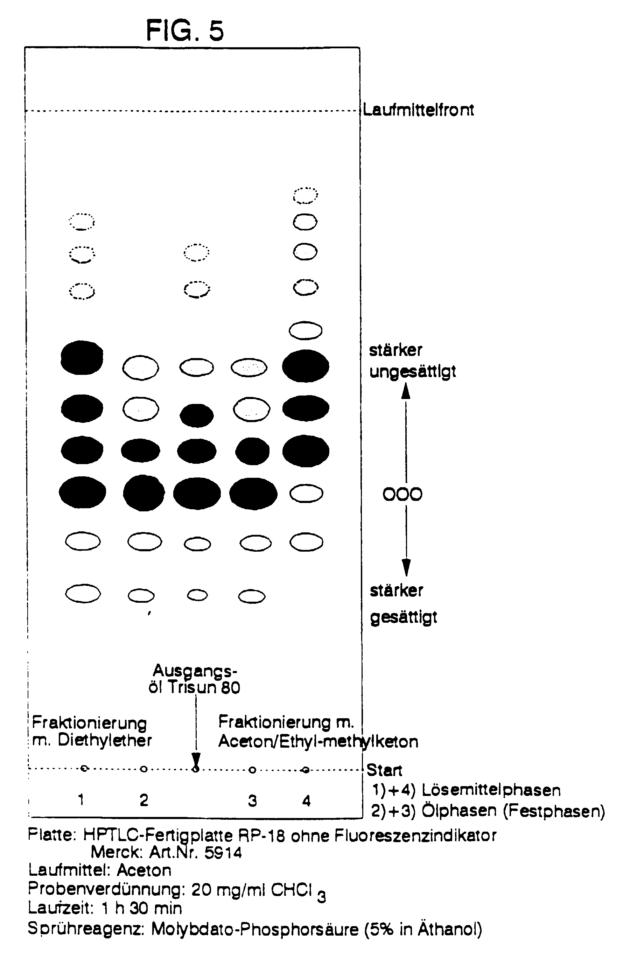

Das Chromatogramm (Fig. 5, Proben 3 und 4) zeigt, daß die Lösemittelphase 4 die mehrfach ungesättigten Fettsäuren enthaltende Triglyceride deutlich angereichert enthält. Im Vergleich zur Fraktionierung mit Diethylether (Proben 1 und 2) sind hier hauptsächlich die Triglyceride OOL, OOE, LOL in der Lösemittelphase 4 enthalten, während bei der Diethylether-Lösemittelphase 1 auch OOO mit enthalten ist. Mit der Aceton/Ethyl-methylketon-Mischung ist also ein gezielteres "Abschneiden" der mehrfach ungesättigte Fettsäuren enthaltenden Triglyceride möglich. Die Ölphase 3 zeigt eine im Vergleich zum Ausgangsöl verminderte Intensität der oberen Flecken (wahrscheinlich OOE + LOL).

### Beispiel 4

### Fraktionierung von Sonnenblumenöl mit Diethylether

### Lösemittel: Diethylether

### Fraktionierung

10 g Trisun 80 - Öl werden mit 10 g Diethylether vermischt (1:1 (Massenanteile)). Bei Raumtemperatur (20°C) löst sich das Öl im Ether. Die Mischung wird über Nacht (18 Stdn.) im Tiefkühlschrank bei -27°C aufbewahrt. Die grobkörnige, weiße Festphase wird über eine vorgekühlte Glasfritte durch Absaugen mit einer Wasserstrahlpumpe abgetrennt. Bei Raumtemperatur verflüssigt sich die Festphase wieder. Bei beiden Phasen wird nun der Diethylether am Rotationsverdampfer entfernt.

### Ausbeuten:

Ölphase (= Festphase): 7,0 g

Öl in der Lösemittelphase (= Flüssigphase): 2,9 g

### Auswertung:

Das Chromatogramm (Fig. 5, Proben 1 und 2) zeigt, daß die Lösemittelphase 1 im Vergleich zur Ölphase 2 mehrfach ungesättigte Fettsäuren enthaltende Triglyceride angereichert enthält.

### Beispiel 5

### Fraktionierung von Sonnenblumenöl mit Aceton / Diethylether

### Verwendetes Lösemittel:

Aceton : Diethylether = 9:1 (Volumenanteile)

### Fraktionierung

5 g Trisun 80 - Öl werden mit 15 g des Lösemittels vermischt (1:3 (Massenanteile)). Bei Raumtemperatur liegt eine flüssige Phase vor. Die Mischung wird über Nacht im Tiefkühlschrank bei -27°C aufbewahrt. Die entstandene Festphase wird über eine vorgekühlte Glasfritte durch Absaugen mit einer Wasserstrahlpumpe getrennt. Bei Raumtemperatur verflüssigt sich die Festphase wieder. Bei beiden Phasen wird nun die Lösemittelmischung mit dem Rotationsverdampfer entfernt.

### Ausbeuten:

Öl in der Festphase: 4,2 g

Öl in der Lösemittelphase: 0,5 g

### Auswertung:

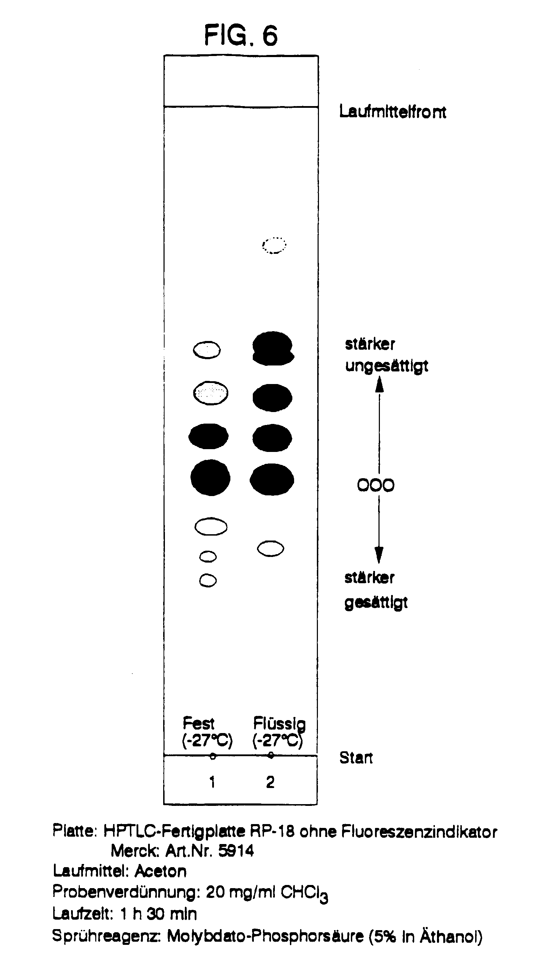

Das Chromatogramm (Fig. 6) zeigt, daß die Lösemittelphase die mehrfach ungesättigten Fettsäuren enthaltende Triglyceride deutlich angereichert enthält. Die Festphase zeigt eine Verringerung dieser Triglyceride, bezogen auf das Ausgangsöl. Eine Fraktionierung mit dieser Lösemittelmischung erweist sich als gut möglich. Im Vergleich zur Fraktionierung mit einem Aceton / Ethyl-methyl-keton-Gemisch ist hier in der Lösemittelphase jedoch mehr Trioleat enthalten (vgl. Probe Nr. 4 in Fig. 5).

Im folgenden seien die Vorteile des erfindungsgemäß erhaltenen Rohstoffs in Derivatisierungsverfahren angegeben:

### a) Einstufige Prozesse

Wie dargelegt, ist der erfindungsgemäß gewonnene Rohstoff dem derzeit in der Oleochemie eingesetzten Rohstoff für Derivatisierungsprozesse an der Ölsäure deutlich überlegen. Bei der Ozonolyse entsteht in erhöhter Ausbeute Azelain- und Pelargonsäure. Nebenprodukte und der Aufwand, diese Nebenprodukte abzutrennen, wie er bei Einsatz des derzeitigen Rohstoffs notwendig ist, entfallen.

Neben der Ozonolyse hat die Überführung der Ölsäure in Hydroxystearinsäure ("Hydroxylierung", Anlagerung von Wasser an die Doppelbindung) derzeit und vor allem in Zukunft entscheidende Bedeutung. Die Hydroxystearinsäuren können in einer Vielzahl von technischen Einsatzfällen, wie beispielsweise bei der Erzeugung von Gleitmitteln in der Kunststoffverarbeitung, eingesetzt werden. Von ihnen ausgehend, können auch neuartige Fettsäurekunststoffe aufgebaut werden.

Erfolgt die Erzeugung der Hydroxystearinsäure aus dem klassischen Rohstoff (ölsäurereiche Talgfraktion bzw. Talgfettsäurefraktion), so ergibt sich ein aufwendiges Trennproblem für die Abtrennung der aus Linol- und Linolensäure zwangsweise auch entstehenden Di- und Trihydroxystearinsäuren.

Darüber hinaus können die mehrfach ungesättigten Fettsäuren bei der Hydroxylierungsreaktion störende Nebenreaktionen auslösen und zu Folgeverbindungen umgesetzt werden, deren Abtrennung äußerst aufwendig und störend ist. So können z. B. Radikalreaktionen, Umlagerungsreaktionen, Autoxidationsreaktionen und vieles andere mehr ablaufen.

Eine weitere bekannte Derivatisierungsreaktion an der Ölsäure ist die Überführung der Ölsäure in die Dihydroxystearinsäure durch Oxidation, wobei als Oxidationsmittel beispielsweise Permanganat, Dichromat, Osmiumtetroxid oder H₂O₂ in Betracht kommen. Die Reaktion kann mit den Triglyceriden selbst oder den entsprechenden Fettsäuren und Fettsäurederivaten durchgeführt werden. Alle diese Oxidationsmittel können auch mit den als Verunreinigungen im klassischen Rohstoff vorhandenen Linol- und Linolensäuren Reaktionen auslösen, so daß auch hier neben der Dihydroxystearinsäure Tetra- und Hexahydroxystearinsäure entstehen können Diese behindern die Isolierung der gewünschten Dihydroxystearinsäure bzw. ihrer Derivate.

Darüber hinaus können auch Nebenreaktionen ausgelöst werden, die für mehrfach ungesättigte Fettsäuren typisch sind, und Folgeverbindungen entstehen, die eine Isolierung der 9,10-Dihydroxystearinsäure, wie sie bei der Oxidation von Ölsäure entsteht, zusätzlich stark behindern. Auf den Mehrverbrauch an Oxidationsmitteln wurde bereits hingewiesen.

Berücksichtigt werden muß weiterhin, daß die Umsetzung bei mehrfach ungesättigten Fettsäuren - anders als bei der Ölsäure als einfach ungesättigter Fettsäure - an den jeweiligen Doppelbindungen unterschiedlich schnell und nicht einheitlich erfolgen kann. Das Spektrum der möglichen, eine Isolierung der gewünschten Ölsäurederivate behindernden und störenden Nebenprodukte wird dadurch ausgesprochen vielfältig.

Im Fall der Verwendung des erfindungsgemäßen Rohstoffs entfallen alle diese Erschwernisse.

Eine weitere, industriell interessante Derivatisierungsreaktion ist die Epoxidierung von Fettsäuredoppelbindungen zu den entsprechenden Epoxyfettsäuren. Wichtigstes industrielles Produkt sind dabei die epoxidierten Öle von solchen Pflanzenölen, die einen hohen Anteil an ungesättigten Fettsäuren enthalten. Eingesetzt werden diese Epoxyöle als Stabilisatoren vor allem in der kunststofferzeugenden Industrie.

Epoxidierte Öle können auch als Grundstoff für die Erzeugung von chemischen Folgeprodukten eingesetzt werden. Dies gilt insbesondere, wenn es gelingt, aus ihnen chemisch einheitliche Verbindungen zu isolieren. Werden klassische, selbst stark ölsäurehaltige Rohstoffe, wie sie aus Talg gewonnen werden, zur Epoxidierung gebracht, so entstehen auch hier Nebenprodukte, die die Weiterverwendung der Epoxytriglyceride, insbesondere zu chemisch einheitlichem Chemierohstoff, stark behindern.

Wenn von unbehandelten High-oleic-Ölen ausgegangen wird, entsteht ein Epoxyöl mit deutlich höherem Epoxyölsäureanteil als bei angereichertem Talg aus Ausgangsmaterial: durch die Anwesenheit von Linol- und Linolensäure im Rohstoff ist es jedoch äußerst heterogen zusammengesetzt. Ähnlich wie bei der Oxidation der Doppelbindungen zu Diolen entsteht bei der Epoxidierung ein bei Raumtemperatur festes Produkt, das jedoch wachsartigen und nicht kristallin rieselfähigen Charakter aufweist. Wird dagegen vom erfindungsgemäßen Ölsäurerohstoff ausgegangen, so wird ein Produkt erhalten, das eindeutig kristallinen Charakter zeigt. Von diesem Produkt ausgehend, lassen sich chemische Folgeverbindungen erheblich vereinfacht herstellen, da solche Nebenreaktionen und Folgereaktionen ausgeschlossen sind, die von Epoxidierungsprodukten der Linol- und Linolensäure ausgelöst werden können. (Hingewiesen sei darauf, daß sowohl Linol- und Linolensäure - anders als die Ölsäure - eine bzw. zwei aktive Methylengruppen enthalten und damit zu Reaktionen fähig sind, die von der Ölsäure ausgehend nicht durchgeführt werden können.)

### b) Mehrstufige Prozesse:

Wird an die Ölsäure Halogenwasserstoff angelagert mit dem Ziel, auf diese Weise durch Umsatz mit Ammoniak zu den entsprechenden Aminosäuren zu kommen, so wird, ausgehend vom erfindungsgemäß hergestellten Rohstoff, über die entsprechenden Ketoverbindungen 9- bzw. 10-Aminostearinsäure als Reaktionsprodukt erhalten. Alle anderen Bestandteile des Rohstoffs reagieren nicht. Sind dagegen Linol- und Linolensäure anwesend, so wird das Spektrum der möglichen Nebenreaktionen derart unübersichtlich, daß eine Abtrennung chemisch einheitlicher Aminosäuren kaum möglich erscheint. Es ist daher verständlich, daß dieser mehrere Stufen umfassende Umsetzungstyp bisher Industriell keine Bedeutung erlangt hat. Gleiches gilt für eine Vielzahl anderer Reaktionen. Beispielhaft soll auf die Analogreaktion zur Erzeugung von Aminocapronsäure, dem Ausgangsmaterial für Nylon, hingewiesen werden.

Ölsäure läßt sich oxidativ in die 9- bzw. 10-Ketostearinsäure überführen. Umgelagert nach Schmidt oder Beckmann, erfolgt dabei eine Spaltung der Kohlenstoffkette der Fettsäure unter Bildung folgender Verbindungen: 1-Aminononan, 1-Aminodekan, Nonansäure, Dekansäure, beide linear aufgebaut und unverzweigt, Dicarbonsäure mit Kettenlängen von C₁₀ und C₉ und lineare Aminosäuren mit endständiger Aminogruppe und Kettenlängen von C₁₀ und C₉. Sämtliche entstehenden Verbindungen können hochwertige Anwendungen einschließen. Die Trennung der entstehenden Verbindungstypen - Amine, Aminosäuren, Monocarbonsäuren, Dicarbonsäuren - ist Stand der Technik.

Wird vom derzeit verfügbaren Rohstoff aus Talg oder von anderen ölsäurereichen, jedoch mehrfach ungesättigte Fettsäuren als Nebenprodukt enthaltenden Stoffen, wie z. B. High-oleic-Ölen, ausgegangen, entsteht ein wesentlich breiteres Spektrum, für das eine Isolierung der entstehenden Folgeprodukte praktisch nicht möglich ist.

Die erfindungsgemäß gewonnene Ölsäurefraktion stellt damit erstmals einen Rohstoff dar, von dem ausgehend, obwohl er selbst chemisch nicht einheitlich zusammengesetzt ist, einheitlich chemische Reaktionen an der Doppelbindung der Ölsäure durchgeführt werden können. Hiermit wird die Ölsäure als Chemiegrundstoff erstmals auf einfache und preiswerte Weise verfügbar.

## Patentansprüche

1. Verfahren zur Herstellung eines von bei der Weiterverarbeitung störenden Bestandteilen befreiten Triglyceridgemisches aus einem Pflanzenöl und/oder einem das unveränderte Kohlenstoffgerüst eines Pflanzenöls enthaltenden Derivat als Ausgangssubstanz, die als solche oder in Lösung abgekühlt und anschließend in eine überwiegend feste und in eine überwiegend flüssige Phase aufgetrennt wird, worauf das Abkühlen und Trennen ggf. wiederholt werden,
**dadurch gekennzeichnet,**
daß man
a) als Ausgangssubstanz ein Pflanzenöl einsetzt, in dem eine bestimmte reaktive Fettsäure im Fettsäuremuster mit einem Gehalt von wenigstens etwa 80% dominiert, daß
b) die Abkühltemperatur eines ersten oder einzigen Abkühlschritts so eingestellt wird, daß die besonders schwer erstarrenden bzw. gut löslichen, nicht einheitlich zusammengesetzten Triglyceride der Ausgangssubstanz, die in der Regel mehr reaktive Gruppen enthalten, zumindest im wesentlichen flüssig bzw. in Lösung bleiben und die Fraktion der die bestimmte, dominierende reaktive Fettsäure enthaltenden Triglyceride zusammen mit den in Bezug hierauf leichter erstarrenden bzw. weniger löslichen und weniger reaktive Gruppen tragenden Triyglyceriden im wesentlichen ausfallen, daß
c) ggf. die nach dem ersten Trennschritt erhaltene feste Phase anschließend gelöst und in einem zweiten Abkühlschritt auf eine im Vergleich zur Temperatur in Schritt b) höhere Temperatur abgekühlt wird, bei der die leichter erstarrenden bzw. weniger löslichen und weniger reaktive Gruppen tragenden, nicht einheitlich zusammengesetzten Triglyceride zumindest im wesentlichen ausfallen und die Fraktion der Triglyceride der bestimmten, dominierenden reaktiven Fettsäure im wesentlichen flüssig bzw. in Lösung bleibt, und daß
d) in jedem Fall die zuletzt erhaltene feste und flüssige Phase getrennt und verwertet werden, ggf. nachdem der erste und bzw. oder die beiden Abkühl- und Trennschritte ein- oder mehrmals wiederholt wurden.

2. Verfahren zur Herstellung von zumindest technisch reinem, nur einen Fettsäuretyp enthaltendem Triglycerid aus einem Pflanzenöl und/oder einem das unveränderte Kohlenstoffgerüst eines Pflanzenöls enthaltenden Derivat als Ausgangssubstanz, die als solche oder in Lösung abgekühlt und anschließend in eine überwiegend feste und in eine überwiegend flüssige Phase aufgetrennt wird, worauf das Abkühlen und Trennen ggf. wiederholt werden,
**dadurch gekennzeichnet**,
daß man
a) als Ausgangssubstanz ein Pflanzenöl einsetzt, in dem eine bestimmte reaktive Fettsäure im Fettsäuremuster mit einem Gehalt von wenigstens etwa 80% dominiert, daß
b) die Abkühltemperatur eines ersten Abkühlschritts so eingestellt wird, daß die besonders schwer erstarrenden bzw. gut löslichen, nicht einheitlich zusammengesetzten Triglyceride der Ausgangssubstanz, die in der Regel mehr reaktive Gruppen enthalten, zumindest im wesentlichen flüssig bzw. in Lösung bleiben und die Fraktion der die bestimmte, dominierende reaktive Fettsäure enthaltenden Triglyceride zusammen mit den in Bezug hierauf leichter erstarrenden bzw. weniger löslichen und weniger reaktive Gruppen tragenden Triyglyceriden im wesentlichen ausfallen, daß
c) die nach dem ersten Trennschritt erhaltene feste Phase anschließend gelöst und in einem zweiten Abkühlschritt auf eine im Vergleich zur Temperatur in Schritt b) höhere Temperatur abgekühlt wird, bei der die leichter erstarrenden bzw. weniger löslichen und weniger reaktive Gruppen tragenden, nicht einheitlich zusammengesetzten Triglyceride zumindest im wesentlichen ausfallen und die Fraktion der Triglyceride der bestimmten, dominierenden reaktiven Fettsäure im wesentlichen flüssig bzw. in Lösung bleibt, und daß
d) die zuletzt erhaltene feste und flüssige Phase getrennt und verwertet werden, ggf. nachdem die beiden Abkühl- und Trennschritte ein- oder mehrmals wiederholt wurden.

3. Verfahren nach Anspruch 1 oder 2,
dadurch gekennzeichnet,
daß auch die flüssige Phase des ersten ggf. wiederholten Trennschritts abgetrennt und verwertet wird.

## Claims

1. Process for the preparation of a triglyceride mixture freed from components which impair further processing, from a vegetable oil and/or a derivative containing the unchanged carbon framework of a vegetable oil as the starting material, which is cooled as such or in solution and then separated into one predominantly solid phase and one predominantly liquid phase, whereupon the cooling and separation are optionally repeated,
characterised in that
a) a vegetable oil is used as starting material in which a given reactive fatty acid predominates in the fatty acid pattern with a content of at least about 80%,
b) the cooling temperature in the first or only cooling step is adjusted so that those triglycerides of non-uniform composition present in the starting material which are particularly difficult to solidify and/or are readily soluble, which as a rule contain more reactive groups, remain at least substantially liquid and/or in solution, and the fraction of the triglycerides containing the given, predominating reactive fatty acid together with the triglycerides which in relation thereto solidify more readily and/or are less soluble and carry fewer reactive groups substantially separate out,
c) optionally the solid phase obtained after the first separation step is then dissolved and is cooled in a second cooling step to a higher temperature than the temperature in step b), when the triglycerides which solidify more readily and/or are less soluble and which carry fewer reactive groups and are not of uniform composition at least substantially separate out, and the fraction of the triglycerides of the given, predominating reactive fatty acid remains substantially liquid and/or in solution, and
d) in any case the solid and liquid phases which are finally obtained are separated and used optionally after the first and/or after the two cooling and separation steps have been repeated one or more times.

2. Process for the preparation of a triglyceride which is at least technically pure and contains only one type of fatty acid, from a vegetable oil and/or a derivative containing the unchanged carbon framework of a vegetable oil as the starting material, which is cooled as such or in solution and then separated into one predominantly solid phase and one predominantly liquid phase, whereupon the cooling and separation are optionally repeated,
characterised in that
a) a vegetable oil is used as starting material in which a given reactive fatty acid predominates in the fatty acid pattern with a content of at least about 80%,
b) the cooling temperature in a first cooling step is adjusted so that those triglycerides of non-uniform composition present in the starting material which are particularly difficult to solidify and/or are readily soluble, which as a rule contain more reactive groups, remain at least substantially liquid and/or in solution, and the fraction of the triglycerides containing the given, predominating reactive fatty acid together with the triglycerides which in relation thereto solidify more readily and/or are less soluble and carry fewer reactive groups substantially separate out,
c) the solid phase obtained after the first separation step is then dissolved and is cooled in a second cooling step to a higher temperature than the temperature in step b), when the triglycerides which solidify more readily and/or are less soluble and which carry fewer reactive groups and are not of uniform composition at least substantially separate out, and the fraction of the triglycerides of the given, predominantly reactive fatty acid remains substantially liquid or in solution, and
d) the solid and liquid phases which are finally obtained are separated and used if desired after the two cooling and separation steps have been repeated one or more times.

3. Process in accordance with Claim 1 or 2, characterised in that the liquid phase from the first, optionally repeated, separation step is also separated and used.

## Revendications

1. Procédé de préparation d'un mélange de triglycérides dépourvu de composants gênants lors du traitement ultérieur, à partir d'une huile végétale et/ou d'un dérivé contenant le squelette carboné non modifié d'une huile végétale, comme substance de départ, qui telle quelle ou en solution, est refroidie et ensuite séparée en une phase principalement liquide et en une phase principalement solide, à la suite de quoi on répète le cas échéant le refroidissement et la séparation,
caractérisé en ce que
a) on utilise comme substance de départ une huile végétale dans laquelle un acide gras réactif déterminé est prépondérant, avec une teneur d'au moins environ 80 %, dans l'échantillon d'acides gras,
b) la température de refroidissement d'une première ou unique étape de refroidissement est réglée de sorte que les triglycérides se solidifiant particulièrement difficilement ou les triglycérides facilement solubles, de composition hétérogène, de la substance de départ, qui en général contiennent des groupes plus réactifs, restent au moins pour l'essentiel liquides ou en solution et la fraction des triglycérides contenant l'acide gras réactif déterminé prépondérant, conjointement avec les triglycérides se solidifiant plus aisément ou portant des groupes moins solubles et moins réactifs, par rapport à ceux-ci, précipitent en grande partie, en ce que
c) le cas échéant la phase solide obtenue après la première étape de séparation est ensuite dissoute et, dans une deuxième étape de refroidissement, refroidie à une température supérieure par comparaison à la température dans l'étape b), à laquelle les triglycérides de composition hétérogène, se solidifiant plus aisément ou portant des groupes moins solubles et moins réactifs précipitent au moins en grande partie et la fraction des triglycérides de l'acide gras réactif déterminé, prépondérant, reste pour l'essentiel liquide ou en solution, et en ce que
d) en tout cas, la phase solide et la phase liquide obtenues en dernier lieu sont séparées et exploitées, le cas échéant après que la première et/ou les deux étapes de refroidissement et de séparation ont été répétées une ou plusieurs fois.

2. Procédé de préparation d'un triglycéride au moins industriellement pur, ne contenant qu'un type d'acide gras, à partir d'une huile végétale et/ou d'un dérivé contenant le squelette carboné non modifié d'une huile végétale, comme substance de départ, qui, telle quelle ou en solution, est refroidie et ensuite séparée en une phase principalement liquide et en une phase principalement solide, à la suite de quoi on répète le cas échéant le refroidissement et la séparation,
caractérisé en ce que
a) on utilise comme substance de départ une huile végétale dans laquelle un acide gras réactif déterminé est prépondérant, avec une teneur d'au moins environ 80 %, dans l'échantillon d'acides gras,
b) la température de refroidissement d'une première étape de refroidissement est réglée de sorte que les triglycérides se solidifiant particulièrement difficilement ou les triglycérides facilement solubles, de composition hétérogène, de la substance de départ, qui en général contiennent des groupes plus réactifs, restent au moins pour l'essentiel liquides ou en solution et la fraction des triglycérides contenant l'acide gras réactif déterminé prépondérant, conjointement avec les triglycérides se solidifiant plus aisément ou portant des groupes moins solubles et moins réactifs, par rapport à ceux-ci, précipitent en grande partie, en ce que
c) la phase solide obtenue après la première étape de séparation est ensuite dissoute et, dans une deuxième étape de refroidissement, refroidie à une température supérieure par comparaison à la température dans l'étape b), à laquelle les triglycérides de composition hétérogène, se solidifiant plus aisément ou portant des groupes moins solubles et moins réactifs précipitent au moins en grande partie et la fraction des triglycérides de l'acide gras réactif déterminé, prépondérant, reste pour l'essentiel liquide ou en solution, et en ce que
d) la phase solide et la phase liquide obtenues en dernier lieu sont séparées et exploitées, le cas échéant, après que la première et/ou les deux étapes de refroidissement et de séparation ont été répétées une ou plusieurs fois.

3. Procédé selon la revendication 1 ou 2,
caractérisé en ce que la phase liquide de la première étape de séparation répétée le cas échéant est également séparée et exploitée.
